# FASCICULE DE BREVET EUROPEEN

(11) **EP 3 829 786 B1**
(45) Date de publication et mention de la délivrance du brevet: **08.01.2025**
(21) Numéro de dépôt: 19774163.0
(22) Date de dépôt: 02.09.2019
(51) Int. Cl.: B08B 9/043, A61B 1/12, A61M 1/00, F41A 29/02, A61B 90/70

(54) **ÉLÉMENT FILAIRE TEXTILE POUR LE TRAITEMENT DE L'INTÉRIEUR D'UN TUBE COMPORTANT DES PARTIES RIGIDE ET SOUPLE**
TEXTILES FADENELEMENT ZUR BEHANDLUNG DER INNENSEITE EINES SCHLAUCHS MIT STARREN UND FLEXIBLEN TEILEN
TEXTILE THREAD ELEMENT FOR TREATING THE INSIDE OF A TUBE COMPRISING RIGID AND FLEXIBLE PORTIONS

(30) Priorité: 06.09.2018 FR 1858019
(43) Date de publication de la demande: 09.06.2021
(73) Titulaire: Commissariat à l'Energie Atomique et aux Energies Alternatives, 75015 Paris (FR); STN, 59560 Comines (FR)
(72) Inventeur: GUYON, Frédérik, 33850 LEOGNAN (FR); NAVARRO, Guillaume, 38610 CESTAS (FR); FAUCHILLE, Mathias, 59560 COMINES (FR)
(74) Mandataire: Brevalex
(86) Numéro de dépôt international: PCT/FR2019/052015
(87) Numéro de publication internationale: WO 2020/049246

(56) Documents cités:
- EP-A1- 0 625 032
- EP-A1- 2 397 066
- EP-A1- 2 918 220
- WO-A1-2007/013196
- DE-A1- 102007 009 949
- US-A- 4 947 880
- US-A- 4 974 615
- US-A- 5 320 117
- US-A- 5 871 589
- US-A1- 2004 187 892
- US-A1- 2016 223 282
- US-A1- 2017 146 313
- US-A1- 2017 216 890
- US-B1- 9 400 152

## Description

### DOMAINE TECHNIQUE

La présente invention se rapporte au domaine général du traitement de l'intérieur de tubes de diamètre millimétrique, ce traitement comprenant notamment le rodage, le polissage et/ou le nettoyage de l'intérieur des tubes.

Plus précisément, l'invention concerne une ficelle comprenant une première partie rigide et une deuxième partie souple, un ensemble comportant une telle ficelle et un tube dans lequel la ficelle est introduite, ainsi qu'un procédé de fabrication d'une telle ficelle.

### ÉTAT DE LA TECHNIQUE ANTÉRIEURE

Dans toute la description, l'expression « traitement de l'inter-tube » désigne une ou des opérations de rodage, polissage et/ou nettoyage de l'intérieur d'un tube de diamètre millimétrique. De plus, par « diamètre millimétrique », on entend un diamètre de l'ordre de quelques dixièmes de millimètres à quelques millimètres.

Le traitement de l'inter-tube de diamètre millimétrique sur de grandes longueurs, typiquement de l'ordre de plusieurs dizaines de centimètres, voire plus d'un mètre, est source de difficultés avec les solutions connues de l'art antérieur. Celles-ci comprennent classiquement : les écouvillons ; des tiges rigides revêtues ou non de matériau adéquat ; une ficelle ou étoffe frottante de diamètre proche, voire légèrement supérieur, à l'inter-tube, préalablement introduite à l'aide d'un tire-fil.

Concernant les écouvillons, leur tête est soit en matériau métallique, ce qui permet de décoller efficacement les dépôts salissants mais entraîne un risque de rayures du tube, soit en matériau polymère, qui ne raye pas le tube mais qui n'a qu'une efficacité très limitée face aux salissures adhérentes. De plus, le matériau polymère est fragile et s'use rapidement au point de ne plus pouvoir assurer le frottement nécessaire aux divers traitements à l'intérieur du tube. Par ailleurs, la tige des écouvillons est en matériau métallique, ce qui est nécessaire à la raideur et à la transmission du couple de torsion dans le mouvement de rotation de l'écouvillon. Cette tige, qui peut être en contact avec le tube de grande longueur, peut donc aussi rayer l'intérieur du tube.

Concernant les tiges rigides revêtues ou non de matériau adéquat, ce sont des tiges suffisamment rigides pour supporter les efforts de torsion et de compression/traction liés aux mouvements de va-et-vient et de rotation nécessaires aux opérations de traitement de l'inter-tube, autrement dit aux opérations de rodage, de polissage et/ou de nettoyage. A titre d'exemples, les matériaux utilisés pour de telles tiges rigides peuvent comprendre le bois ou le métal. Les tiges peuvent par exemple être des tiges métalliques monobrin ou multibrins revêtues de matériau adéquat, tel qu'un matériau abrasif pour le rodage ou polissage, ou un matériau doux pour le nettoyage. A noter également que, pour des diamètres de l'inter-tube de l'ordre du millimètre, il existe des tiges ou des câbles métalliques capables de transmettre ces efforts, mais cette solution est adaptée pour un rodage ou polissage grossier sans permettre donc un rodage ou polissage fin, ni le cas échéant un nettoyage sans détérioration de l'état de surface interne du tube.

Concernant la ficelle ou étoffe frottante, il s'agit d'une matière textile, synthétique ou naturelle, qui est tirée en force à l'intérieur du tube. Une des difficultés d'utilisation repose sur l'introduction de cette ficelle de diamètre nominal nécessairement proche, voire légèrement supérieur, au diamètre interne du tube. Pour ce faire, on utilise un tire-fil de petit diamètre, en matière polymère ou métallique gainée pour ne pas rayer le tube. Le tire-fil est alors passé en premier, puis une liaison mécanique est réalisée avec la matière textile. Enfin, par traction sur le tire-fil, on amène progressivement la matière textile au travers du tube. Cette solution fonctionne pour des tubes de quelques millimètres de diamètre interne, par exemple pour un diamètre interne de 2,5 mm ou plus. Toutefois, elle s'avère très délicate à utiliser pour des longs tubes avec des diamètres internes plus faibles. Par ailleurs, il faut noter qu'il existe des tire-fils et des matières textiles suffisamment résistants pour supporter les efforts, mais toute la difficulté réside dans la fragilité du raccord entre tire-fil et matière textile : en particulier, soit ce raccord impose un diamètre trop important ; soit il impose des rayons de courbure trop faibles qui ne tiennent pas les efforts ; soit encore il impose un tire-fil et/ou un matériau textile de diamètre trop petit pour être fonctionnel ou pour supporter les efforts de traction.

Les documents EP0625032 A1, divulguant le préambule de la revendication 1, et US2004/187892 A1 font partie de l'état de la technique.

### EXPOSÉ DE L'INVENTION

L'invention a ainsi pour but de remédier au moins partiellement aux besoins mentionnés précédemment et aux inconvénients relatifs aux réalisations de l'art antérieur.

L'invention a ainsi pour objet, selon l'un de ses aspects, une ficelle pour le traitement d'un tube, notamment un tube de diamètre millimétrique, caractérisé en ce qu'elle comporte :
- une première partie, dite « partie rigide », formant tire-fil pour l'introduction et la traction avant de la ficelle dans l'intérieur du tube,
- une deuxième partie, dite « partie souple », formant fil souple pour le traitement de l'intérieur du tube,
la rigidité de la première partie étant supérieure à celle de la deuxième partie, et la première partie et la deuxième partie étant réalisées dans le ou les mêmes matériaux et dans la continuité l'une de l'autre.

Par « rigide », il faut comprendre que la première partie peut être rigide ou semi-rigide.

De plus, il est à noter que la deuxième partie peut éventuellement aussi servir pour la traction avant de la ficelle dans l'intérieur du tube, en particulier en cas d'introduction totale dans l'intérieur du tube et de dépassement de part et d'autre du tube.

D'après l'invention les première et deuxième parties sont réalisées dans la continuité l'une de l'autre. Autrement dit, il n'existe pas de discontinuité entre les deux parties et aucune liaison mécanique n'est nécessaire pour les relier entre elles. Les première et deuxième parties sont d'après l'invention formées d'un seul tenant. Elles sont d'après l'invention reliées entre elles de manière continue, avec absence de toute jonction. La deuxième partie comporte des fils métalliques.

Grâce à l'invention, il peut être possible de réaliser la fonctionnalité de tire-fil et la fonctionnalité de traitement de l'inter-tube par le biais de matière(s) textile(s) identique(s) supprimant ainsi le besoin d'une liaison mécanique inhérente aux solutions hétérogènes de l'art antérieur prévoyant une séparation initiale entre tire-fil et matière textile.

La ficelle selon l'invention peut en outre comporter l'une ou plusieurs des caractéristiques suivantes prises isolément ou suivant toutes combinaisons techniques possibles.

La deuxième partie peut comporter, sur sa longueur, une ou plusieurs variations géométriques en surface, notamment une ou plusieurs aspérités sur sa surface, par exemple des reliefs et/ou creux, utilisées pour le traitement de l'intérieur du tube.

La première partie peut avantageusement être, sur sa longueur, sensiblement uniforme.

Par ailleurs, la ficelle selon l'invention peut être réalisée au moins partiellement, notamment totalement, à partir de fibres thermodurcissables.

La ficelle peut comporter des fils dédiés à des fonctions spécifiques. En particulier, la deuxième partie peut comporter des fils dédiés à des fonctions spécifiques choisis parmi des fibres d'absorption de fluides et/ou des fibres d'abrasion de l'intérieur du tube entre autres, qui sont avantageusement non thermodurcissables.

Par ailleurs, la ficelle peut comporter en outre une troisième partie, dite « partie souple ou rigide », formant fil de préhension pour la traction arrière de la ficelle dans l'intérieur du tube. La troisième partie et la deuxième partie peuvent être réalisées dans le ou les mêmes matériaux et dans la continuité l'une de l'autre.

De façon avantageuse, les troisième, deuxième et première parties sont réalisées dans la continuité l'une de l'autre. Autrement dit, il n'existe pas de discontinuité entre les trois parties et aucune liaison mécanique n'est nécessaire pour les relier entre elles. Les première, deuxième et troisième parties sont avantageusement formées d'un seul tenant.

De plus, seule la deuxième partie peut comporter un revêtement abrasif.

De façon avantageuse, lorsque la ficelle comporte uniquement une première partie et une deuxième partie, autrement dit aucune troisième partie, la deuxième partie peut former fil de préhension pour la traction arrière de la ficelle dans l'intérieur du tube. Lorsque la ficelle comporte des première, deuxième et troisième parties, la troisième partie est la partie préférentielle pour la traction arrière de la ficelle.

Le cas échéant, dans cette deuxième configuration, la deuxième partie peut aussi être utilisée pour la traction arrière.

En outre, l'invention a encore pour objet, selon un autre de ses aspects, un ensemble, caractérisé en ce qu'il comporte :
- une ficelle telle que définie précédemment,
- un tube, notamment un tube de diamètre millimétrique, dans lequel est introduite la ficelle pour le traitement de l'intérieur du tube.

La longueur de la ficelle peut préférentiellement être supérieure à la longueur du tube.

En outre, la longueur de la première partie rigide peut avantageusement être supérieure à la longueur du tube.

Par ailleurs, l'invention a également pour objet, selon un autre de ses aspects, un procédé de fabrication d'une ficelle telle que définie précédemment, comportant les étapes suivantes :
- tressage, notamment en continu, de filaments textiles pour former la ficelle,
- chauffage, notamment par intermittence, de la première partie pour la rendre rigide.

Avantageusement, lorsque la ficelle comporte au moins partiellement, notamment totalement, des fibres thermodurcissables, le procédé selon l'invention peut comporter l'étape consistant à thermodurcir, notamment par chauffage intermittent, la première partie pour la rendre rigide.

### BRÈVE DESCRIPTION DES DESSINS

L'invention pourra être mieux comprise à la lecture de la description détaillée qui va suivre, d'exemples de mise en œuvre non limitatifs de celle-ci, ainsi qu'à l'examen des figures, schématiques et partielles, du dessin annexé, sur lequel :
- la figure 1 illustre un exemple de ficelle conforme à l'invention,
- la figure 2 illustre un exemple d'ensemble comportant un tube de diamètre millimétrique et une ficelle conforme à l'invention, telle que celle de la figure 1,
- la figure 3 illustre un procédé de fabrication d'une ficelle conforme à l'invention, telle que celle de la figure 1, et
- la figure 4 illustre un autre exemple de ficelle conforme à l'invention.

Les différentes parties représentées sur ces figures ne le sont pas nécessairement selon une échelle uniforme, pour rendre celles-ci plus lisibles.

### EXPOSÉ DÉTAILLÉ DE MODES DE RÉALISATION PARTICULIERS

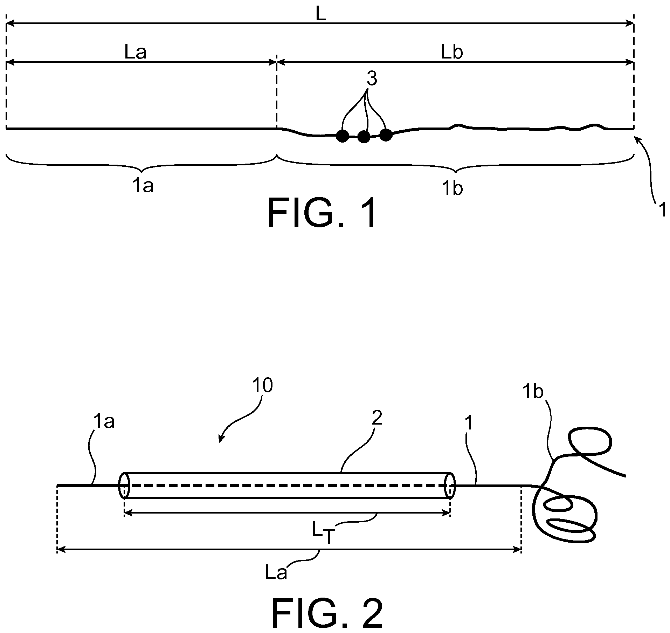

On va décrire ci-après, en référence aux figures 1 à 3, un exemple de ficelle 1 conforme à l'invention pour le traitement de l'intérieur d'un tube 2 de diamètre millimétrique.

Cette ficelle 1 se présente généralement sous la forme d'une ficelle 1 telle que représentée sur la figure 1.

Conformément à l'invention, la ficelle 1 comporte une première partie 1a dite « partie rigide », et une deuxième partie 1b dite « partie souple ».

La ficelle 1 est homogène car les parties rigide 1a et souple 1b sont réalisées dans le même matériau ou les mêmes matériaux, et sont dans la continuité l'une de l'autre. Autrement dit, hormis sur le plan de la rigidité et éventuellement sur le plan de variations géométriques, il n'y a pas de différence entre les parties rigide 1a et souple 1b. Elles sont réalisées d'un seul tenant et définissent ensemble la ficelle 1.

La première partie rigide 1a est plus particulièrement rigide ou semi-rigide. Elle est qualifiée de « rigide » car elle présente une rigidité plus importante que la deuxième partie 1b dite « souple ».

Plus précisément, la ficelle 1 a ainsi une double fonctionnalité, à savoir celle de tire-fil et celle de traitement de l'inter-tube, comme expliqué précédemment dans la partie relative à l'état de la technique antérieur.

Ainsi, la partie rigide 1a présente une rigidité telle que celle utilisée habituellement pour un tire-fil utilisé pour le principe des ficelles ou étoffes frottantes. Cette partie rigide 1a permet l'introduction de la ficelle 1, et notamment de la partie souple 1b, dans le tube 2.

La partie souple 1b présente une souplesse telle que celle utilisée habituellement pour une ficelle ou étoffe frottante classique. Cette partie souple 1b permet le traitement de l'intérieur du tube 2.

La figure 2 illustre l'insertion de la ficelle 1 de la figure 1 dans le tube 2 de diamètre millimétrique.

Plus particulièrement, il peut s'agir d'un tube 2 d'un diamètre interne d'environ 1 mm et d'une longueur L_{T} d'environ 1500 mm.

De façon avantageuse, la longueur L de la ficelle 1 est supérieure à la longueur L_{T} du tube 2. Plus précisément encore, la longueur La de la première partie rigide 1a est supérieure à la longueur L_{T} du tube 2. De cette façon, la partie rigide 1a est entièrement introduite dans le tube 2, une portion de celle-ci dépassant du tube 2 à chacune de ses extrémités comme visible sur la figure 2. Alors, la partie souple 1b de la ficelle 2, visible à droite sur la figure 2, à l'extérieur du tube 2, est prête à être tirée dans le tube 2 pour en permettre le traitement.

Par ailleurs, comme visible sur la figure 1, la partie rigide 1a présente un aspect, le long de sa longueur La, sensiblement uniforme de sorte à faciliter son introduction dans le tube 2 et à ne pas endommager l'intérieur du tube 2.

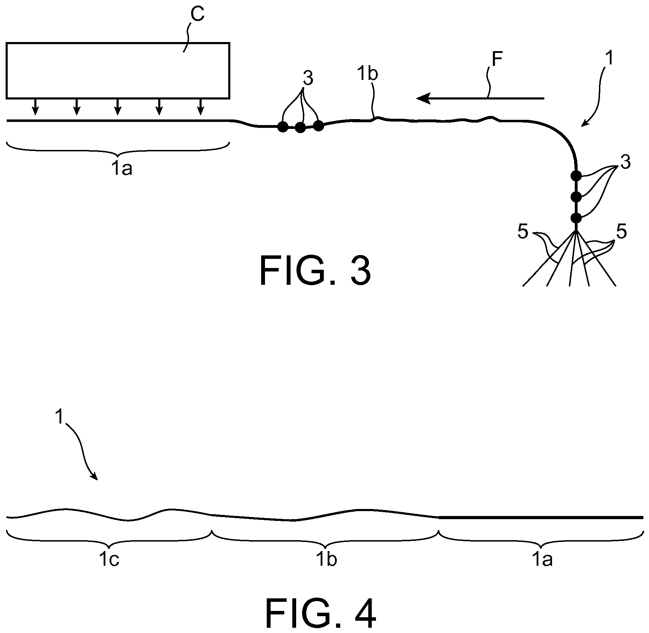

Au contraire, comme visible sur les figures 1 et 3, la partie souple 1b de la ficelle 1 peut comporter, le long de sa longueur Lb, des variations géométriques 3 en surface, ici par exemple des reliefs 3 sous forme sphérique, utilisées pour le traitement de l'intérieur du tube 2. Plus précisément, la ficelle 1 comporte des modifications ponctuelles de géométrie pour fonctionnaliser la partie souple 1b dédiée au traitement de l'inter-tube, par exemple des aspérités de surface, notamment des surépaisseurs, des abrasifs et/ou des cavités pour par exemple venir frotter la paroi interne du tube 2 ou encore maintenir des fluides nettoyants.

En outre, la ficelle 1 est avantageusement réalisée avec des fibres thermodurcissables afin de permettre la formation de la partie rigide 1a.

Plus précisément, on réalise la ficelle 1, assurant les deux fonctions de tire-fil et de traitement de l'inter-tube, par thermodurcissement d'une partie de celle-ci pour former la partie rigide 1a, sans ainsi avoir besoin d'un raccord avec l'autre partie formant la partie souple 1b.

La figure 3 illustre schématiquement un principe de fabrication de la ficelle 1 conforme à l'invention.

La ficelle 1 est ainsi formée, entièrement ou partiellement, par un procédé de tressage classique en continu à partir de fibres thermodurcissables, la flèche F représentant le sens de défilement sur la figure 3.

Ainsi, des filaments textiles 5 sont tressés pour former la ficelle 1 selon le sens de la flèche F sur la figure 3.

La partie rigide 1a, dédiée à la fonction de tire-fil, est constituée par thermodurcissement sur une longueur La égale au minimum à la longueur L_{T} du tube 2 à traiter complétée d'une sur-longueur pour la préhension et l'insertion, par chauffage intermittent C de la partie de la ficelle 1 formée en premier dans le procédé de tressage.

L'autre partie, destinée à former la partie souple 1b, n'est pas chauffée pour maintenir la souplesse mais est pourvue de variations géométriques 3 destinées à améliorer le traitement de l'inter-tube.

L'opération de thermodurcissement peut être réalisée lors du défilement de la ficelle 1 en sortie de l'opération de tressage, comme illustré sur la figure 3, ou encore dans un second temps, après une éventuelle découpe en longueurs multiples de la longueur d'utilisation finale de la ficelle 1.

Par ailleurs, la ficelle 1 peut également comporter, en plus des fibres thermodurcissables, d'autres types de fibres pour conférer d'autres propriétés à la ficelle 1, par exemple des fibres d'absorption ou de retenue de fluides nettoyants et/ou des fibres d'abrasion de l'intérieur du tube 2 et/ou des fibres résistantes pour le traitement de l'inter-tube.

La figure 4 illustre par ailleurs un autre exemple de ficelle 1 conforme à l'invention.

Dans cet exemple, comme précédemment, la ficelle 1 comporte une première partie rigide 1a, et une deuxième partie souple 1b. La partie rigide 1a permet l'insertion et la traction avant. La partie souple 1b permet le traitement de l'intérieur du tube 2.

Cette partie souple 1b peut avoir une première configuration semblable à celle décrite précédemment, pouvant ou non comporter des variations de géométrie le long de celle-ci et pouvant comporter des fils dédiés à des fonctions spécifiques choisis parmi des fibres d'absorption de fluides et/ou des fibres d'abrasion de l'intérieur du tube 2 et/ou des fils métalliques, ceux-ci étant alors avantageusement non thermodurcissables. Elle peut également avoir une deuxième configuration, non exclusive de la première, dans laquelle elle seule est revêtue d'un revêtement abrasif.

Par ailleurs, dans cet exemple, la ficelle 1 comporte également une troisième partie 1c, dite « partie souple ou rigide », formant fil de préhension pour la traction arrière de l'élément filaire 1 dans l'intérieur du tube 2.

Cette troisième partie 1c peut être souple et ainsi constituer une partie spécifique, ou bien être semi-rigide constituant le début de la section suivante.

Bien entendu, l'invention n'est pas limitée aux exemples de réalisation qui viennent d'être décrits. Diverses modifications peuvent y être apportées par l'homme du métier, sans sortir du cadre des revendications.

## Revendications

1. Ficelle (1) pour le traitement d'un tube (2), comportant :
- une première partie (1a), dite « partie rigide », formant tire-fil pour l'introduction et la traction avant de la ficelle (1) dans l'intérieur du tube (2),
- une deuxième partie (1b), dite « partie souple », formant fil souple pour le traitement de l'intérieur du tube (2),
la rigidité de la première partie (1a) étant supérieure à celle de la deuxième partie (1b), et la première partie (1a) et la deuxième partie (1b) étant réalisées dans le ou les mêmes matériaux,
les première (1a) et deuxième (1b) parties sont réalisées dans la continuité l'une de l'autre et d'un seul tenant, étant reliées entre elles de manière continue, avec absence de toute jonction, **caractérisé en ce que**
la deuxième partie (1b) comporte des fils métalliques.

2. Ficelle selon la revendication 1, **caractérisée en ce que** la deuxième partie (1b) comporte, sur sa longueur (Lb), une ou plusieurs variations géométriques (3) en surface utilisées pour le traitement de l'intérieur du tube (2).

3. Ficelle selon la revendication 1 ou 2, **caractérisée en ce que** la première partie (1a) est, sur sa longueur (La), sensiblement uniforme.

4. Ficelle selon l'une des revendications précédentes, **caractérisé en ce qu'**elle est réalisée au moins partiellement à partir de fibres thermodurcissables.

5. Ficelle selon l'une quelconque des revendications précédentes, **caractérisée en ce que** la deuxième partie (1b) comporte également des fils dédiés à des fonctions spécifiques choisis parmi des fibres d'absorption de fluides et/ou des fibres d'abrasion de l'intérieur du tube (2).

6. Ficelle selon l'une quelconque des revendications précédentes, **caractérisée en ce qu'**elle comporte :
- une troisième partie (1c), dite « partie souple ou rigide », formant fil de préhension pour la traction arrière de la ficelle (1) dans l'intérieur du tube (2), la troisième partie (1c) et la deuxième partie (1b) étant réalisées dans le ou les mêmes matériaux et dans la continuité l'une de l'autre.

7. Ficelle selon l'une quelconque des revendications précédentes, **caractérisée en ce que** seule la deuxième partie (1b) comporte un revêtement abrasif.

8. Ensemble (10), **caractérisé en ce qu'**il comporte :
- une ficelle (1) selon l'une quelconque des revendications précédentes,
- un tube (2), dans lequel est introduit la ficelle (1) pour le traitement de l'intérieur du tube (2).

9. Ensemble selon la revendication 8, **caractérisé en ce que** la longueur (L) de la ficelle (1) est supérieure à la longueur (L_{T}) du tube (2).

10. Ensemble selon la revendication 8 ou 9, **caractérisé en ce que** la longueur (La) de la première partie rigide (1a) est supérieure à la longueur (L_{T}) du tube (2).

11. Procédé de fabrication d'une ficelle (1) selon l'une quelconque des revendications 4 à 7, quand dépendante de la revendication 4, comportant les étapes suivantes :
- tressage de filaments textiles (5) pour former la ficelle (1),
- chauffage de la première partie (1a) pour la rendre rigide.

12. Procédé selon la revendication 11, **caractérisé en ce que**, la ficelle (1) comportant au moins partiellement des fibres thermodurcissables, le procédé comporte l'étape consistant à thermodurcir la première partie (1a) pour la rendre rigide.

## Patentansprüche

1. Textiles Fadenelement (1) zum Behandeln eines Schlauchs (2), **dadurch gekennzeichnet, dass** es umfasst:
- einen ersten Abschnitt (1a), welcher der "starre Abschnitt" genannt wird, der einen Fadenzieher zum Einführen und vorderen Ziehen des Fadenelements (1) innerhalb des Schlauchs (2) bildet,
- einen zweiten Abschnitt (1b), der "flexibler" Abschnitt genannt wird, der einen flexiblen Faden zum Behandeln der Innenseite des Schlauchs (2) bildet,
wobei die Starrheit des ersten Abschnitts (1a) größer als jene des zweiten Abschnitts (1b) ist, und der erste Abschnitt (1a) und der zweite Abschnitt (1b) aus dem gleichen Material hergestellt sind,
der erste (1a) und zweite (1b) Abschnitt aus einem einzigen Stück hergestellt sind, die ohne Übergang durchgängig miteinander verbunden sind, **dadurch gekennzeichnet, dass** der zweite Abschnitt (1b) Metallfäden umfasst.

2. Fadenelement nach Anspruch 1, **dadurch gekennzeichnet, dass** der zweite Abschnitt (1b) über seine Länge (Lb) eine oder mehrere geometrische Variationen (3) an der Oberfläche umfasst, die zum Behandeln der Innenseite eines Schlauchs (2) verwendet werden.

3. Fadenelement nach Anspruch 1 oder 2, **dadurch gekennzeichnet, dass** der erste Abschnitt (1a) über seine Länge (La) im Wesentlichen einheitlich ist.

4. Fadenelement nach einem der vorstehenden Ansprüche, **dadurch gekennzeichnet, dass** es mindestens teilweise, insbesondere vollständig aus wärmehärtbaren Fasern hergestellt ist.

5. Fadenelement nach einem vorstehenden Anspruch, **dadurch gekennzeichnet, dass** der zweite Abschnitt (1b) auch Fäden umfasst, die speziellen Funktionen gewidmet sind, die aus Fluidabsorptionsfasern und/oder Abriebfasern für die Innenseite des Schlauchs (2) ausgewählt sind.

6. Fadenelement nach einem vorstehenden Anspruch, **dadurch gekennzeichnet, dass** es umfasst:
- einen dritten Abschnitt (1c), der "flexibler oder starrer Abschnitt" genannt wird, der einen Greiferfaden für das hintere Ziehen des Fadenelements (1) innerhalb des Schlauchs (2) bildet, wobei der dritte Abschnitt (1c) und der zweite Abschnitt (1b) aus dem gleichen Material oder Materialien hergestellt sind und miteinander zusammenhängen.

7. Fadenelement nach einem vorstehenden Anspruch, **dadurch gekennzeichnet, dass** nur der zweite Abschnitt (1b) eine Abriebbeschichtung umfasst.

8. Einheit (10), **dadurch gekennzeichnet, dass** sie umfasst:
- ein textiles Fadenelement (1) nach einem vorstehenden Anspruch,
- einen Schlauch (2), wobei das Fadenelement zum Behandeln der Innenseite des Schlauchs (2) eingeführt (1) wird.

9. Einheit nach Anspruch 8, **dadurch gekennzeichnet, dass** die Länge (L) des Fadenelements (1) größer als die Länge (L_{T}) des Schlauchs (2) ist.

10. Einheit nach Anspruch 8 oder 9, **dadurch gekennzeichnet, dass** die Länge (La) des ersten starren Abschnitts (1a) größer als die Länge (L_{T}) des Schlauchs (2) ist.

11. Verfahren zum Herstellen eines textilen Fadenelements (1) nach einem der Ansprüche 4 bis 7, wenn von Anspruch 4 abhängig, die folgenden Schritte umfassend:
- Flechten von Textilfilamenten (5), um das Fadenelement (1) zu bilden,
- Erhitzen des ersten Abschnitts (1a), um ihn starr zu machen.

12. Verfahren nach Anspruch 11, **dadurch gekennzeichnet, dass**, das Fadenelement (1) mindestens teilweise, insbesondere vollständig wärmehärtbare Fasern umfasst, wobei das Verfahren den Schritt umfasst, der aus dem Wärmehärten des ersten Abschnitts (1a) besteht, um ihn starr zu machen.

## Claims

1. Textile thread element (1) for treating a tube (2), **characterised in that** it comprises:
- a first portion (1a), called the "rigid portion", forming a thread puller for introducing and front traction of the thread element (1) inside the tube (2),
- a second portion (1b), called "flexible" portion, forming a flexible thread for treating the inside of the tube (2),
the rigidity of the first portion (1a) being greater than that of the second portion (1b), and the first portion (1a) and the second portion (1b) being made from the same material,
the first (1a) and second (1b) portions are made from a single piece, being continuously connected together, with the absence of any junction, **characterized in that** the second portion (1b) comprises metallic threads.

2. Thread element according to claim 1, **characterised in that** the second portion (1b) comprises, over its length (Lb), one or more geometrical variations (3) on the surface used for treating the inside of a tube (2).

3. Thread element according to claim 1 or 2, **characterised in that** the first portion (1a) is, over its length (La), substantially uniform.

4. Thread element according to one of the preceding claims, **characterised in that** it is made at least partially, in particular entirely, from thermosetting fibres.

5. Thread element according to any preceding claim, **characterised in that** the second portion (1b) also comprises threads dedicated to specific functions chosen from fluid absorption fibres and/or abrasion fibres of the inside of the tube (2).

6. Thread element according to any preceding claim, **characterised in that** it comprises:
- a third portion (1c), called "flexible or rigid portion", forming a gripper thread for the rear traction of the thread element (1) inside the tube (2), the third portion (1c) and the second portion (1b) being made from the same material or materials and continuous with each other.

7. Thread element according to any preceding claim, **characterised in that** only the second portion (1b) comprises an abrasive coating.

8. Ensemble (10), **characterised in that** it comprises:
- a textile thread element (1) according to any preceding claim,
- a tube (2), wherein the thread element is introduced (1) for treating the inside of the tube (2).

9. Ensemble according to claim 8, **characterised in that** the length (L) of the thread element (1) is greater than the length (L_{T}) of the tube (2).

10. Ensemble according to claim 8 or 9, **characterised in that** the length (La) of the first rigid portion (1a) is greater than the length (L_{T}) of the tube (2).

11. Method for manufacturing a textile thread element (1) according to any of claims 4 to 7 when depending on claim 4, comprising the following steps:
- braiding of textile filaments (5) to form the thread element (1),
- heating of the first portion (1a) to render it rigid.

12. Method according to claim 11, **characterised in that**, the thread element (1) comprising at least partially, in particular entirely, thermosetting fibres, the method comprising the step consisting in thermosetting the first portion (1a) to render it rigid.
